# EUROPEAN PATENT SPECIFICATION

(11) **EP 2 070 515 B1**
(45) Date of publication and mention of the grant of the patent: **05.07.2017**
(21) Application number: 08013903.3
(22) Date of filing: 04.08.2008
(51) Int. Cl.: A61K 8/73, A61K 8/85, A61K 8/898, A61Q 5/12, A61Q 5/02

(54) **Conditioning composition for keratinic fibres**
Zusammensetzung zum Konditionieren von Keratinfasern
Composition de conditionnement pour des fibres de kératine

(30) Priority: 07.08.2007 EP 07015461
(43) Date of publication of application: 17.06.2009
(73) Proprietor: Kao Germany GmbH, 64297 Darmstadt (DE)
(72) Inventor: Hoffmann, Martin, 64673 Zwingenberg (DE); Arbter, Claudia, 64560 Riedstadt (DE)
(74) Representative: Grit, Mustafa

(56) References cited:
- EP-A- 1 378 227
- WO-A-02/22085
- WO-A-97/04743
- WO-A-97/14406
- US-A1- 2004 247 550
- "LUSTREPLEX((R): Polyquaternium-70 (and) Dipropylene Glycol" CRODA INC. - PERSONAL CARE, [Online] 30 July 2007 (2007-07-30), XP002464459 Retrieved from the Internet: URL:http://www.croda.com> [retrieved on 2008-01-11]

## Description

The present invention relates to a conditioning composition for keratin fibres especially human hair comprising particular cationic polymers and a cationic or cationizable silicone compound.

Conditioning composition for keratin fibres have been known for a long time. They are usually applied after cleansing hair and rinsed off from hair, although leave-in conditioning compositions have recently become available and gains more and more popularity among consumers under so called "easy to use" concept.

The function of a hair conditioner as understood by the consumer is first off all detangle hair and furthermore give hair smoothness, elasticity, volume and body, and shine. In spite of availability of various conditioning compositions, there are still needs for improvements. The need for improvement is especially high for consumers either having fine hair, meaning hair with a smaller diameter, or damaged hair for which a high level of conditioning is needed for smoothing and therewith easing further handling for example combability. On the other hand, when a conditioning composition with high level of conditioning ingredients is used, hair looses its volume and body and often lacks shine and elasticity.

WO02/22085 discloses a concentrated hair conditioner which comprises 0.01-50 weight% of a conditioning agent containing cationic surfactants and polymers, silicone compounds and/or polyalkylene glycols and an aqueous carrier. Present invention starts from the above mentioned problems and provides a conditioning composition which effectively detangles hair without loosing volume and body, and with enhanced smoothness and shine.

The present inventors have surprisingly found out that an aqueous hair conditioning composition comprising at least two cationic polymers, one being a cationic polymer with multiple cationic sites and with multiple ester groups in its molecule and the other cationic polymer is being a cellulose derivative, and a cationic or cationizable silicone improves hair combability, smoothness shine, elasticity and more interestingly hair treated with such a composition has volume and body and excellently manageable.

Accordingly, the first subject of the present invention is an aqueous composition for conditioning keratin fibres especially human hair comprising at least one cationic polymer with multiple cationic sites and with multiple ester groups in its molecule, at least one cationic cellulose derivative and at least one cationic or cationizable silicone compound.

Second subject matter of the present invention is the use of the composition comprising at least one cationic polymer with multiple cationic sites and with multiple ester groups in its molecule, at least one cationic cellulose derivative and at least one cationic silicone compound for conditioning hair.

The third subject matter of the present invention is use of the composition for improving smoothness, shine, elasticity volume and body of hair.

The forth subject matter of the present invention is use of the composition for improving smoothness, shine, elasticity volume and body of damaged hair. With the term damaged hair it is meant that hair is undergone chemical treatments such as oxidative colouring, bleaching and/or permanent shaping or damaged with the effects of the environmental influences.

The fifth subject matter of the present invention is use of the composition for improving smoothness, shine, elasticity volume and body of fine hair.

The cationic polymer preferably comprises at least 4 ester groups, at least 3, preferably 5, more preferably 7 and most preferably 9 cationic sites in form of quaternary ammonium groups and at least 3 alkyl groups with an average chain length of 18 carbon atoms which are one of the alkyl groups of different cationic quaternary ammonium site. A cationic polymer of the kind, and most preferred cationic polymer within the meaning of present invention, is available from Croda under the trade name Lustreplex as dispersed in dipropyleneglycol which is named as Polyquaternium-70 according to INCI.

Compositions of the present invention comprise cationic polymer at a concentration of 0.001 to 5%, preferably 0.005 to 3%, more preferably 0.01 to 2.5% and most preferably 0.05 to 1%, in particular 0.1 to 0.5% by weight calculated to total composition.

Compositions of the present invention comprise at least one cellulosic cationic polymer. The cationic cellulose derivatives are well known with the CTFA names, for example, Polyquaternium-4, Polyquaternium-10, Polyquaternium-24, Polyquaternium-67 and Polyquaternium-72 and commercially available from various suppliers under various trade names.

Preferred cationic cellulose derivatives are Polyquaternium-4, Polyquaternium-10, Polyquaternium-24, Polyquaternium-67 and Polyquaternium-72 and more preferred ones are Polyquaternium-4, Polyquaternium-24, Polyquaternium-67 and Polyquaternium-72. The most preferred are Polyquaternium-24, Polyquaternium-67 and Polyquaternium-72.

Cationic cellulose derivatives are comprised in the compositions of the present invention at a concentration of 0.01 - 2.5%, preferably 0.05 - 2%, more preferably 0.1 - 1.5 and most preferably 0.25 - 1% by weight calculated to total composition.

Interestingly, it has also been observed that weight ratio of the first cationic polymer and the second cationic polymer, cationic cellulose derivative, plays also an important role I the conditioning effects of the composition of the present invention. It has been observed that preferably the weight ratio of the first cationic polymer to the second cationic polymer is in the range of 0.1:1 to 2:1, more preferably 0.1:1 to 1:1, and most preferably 0.25:1 to 0.5:1.

Compositions of the present invention comprise at least cationic or cationizable silicone compound. With the term cationizable silicone compound it is meant that at least one free amino group is present on the molecule which can be either primary, secondary or tertitary amine. The free amino group or groups can be protonated and present a cationic charge.

Cationizable silicone compounds are known with the CTFA name amodimethicone and available from Dow Corning under the trade names, for example, DC 949 emulsion, and DC Q2-8220 and Blesil ADM 802 available from Wacker.

Further cationizable silicone compound suitable for the compositions of the present invention is polysilicone-9 (CTFA adopted name) available under the trade name Elastomer OS from Kao Corporation. In this respect reference is made to the cationic polymers disclosed in EP-A 524 612 and EP-A 640 643. They are quaternized products of graft polymers from organopolysiloxanes and polyethyl oxazolines also suitable for the compositions of the present invention.

Cationic silicone compound which is known with the CTFA adopted name Quaternium-80 and is available under the trade name Abil Quat from Degussa.

Cationizable or cationic silicones are included into the compositions of the present invention at a concentration of 0.01 to 5%, preferably 0.05 to 3%, and more preferably 0.1 to 2.5 and most preferably 0.25 to 2% by weight calculated to total composition.

Compositions of the present invention can be in the form of thickened gels and emulsion. In case that composition is a thickened gel it comprises additionally at least one thickening agent. Such thickening agents are for example, cellulose derivatives such as hydroxyethyl or hydroxymethyl cellulose, further cationic polymers such as polyquaternium-37, and anionic polymers such as acrylate copolymers as long as no compatibility problem raises.

Preferably the composition of the present invention is aqueous oil in water emulsion.

Compositions of the present invention comprise at least one fatty alcohol with an alkyl chain length of 12 to 24 C atoms, preferably 14 to 22 C atoms and more preferably 16 to 22 C atoms. Non-limiting examples are lauryl alcohol, myristyl alcohol, cetyl alcohol, stearyl alcohol and behenyl alcohol. Composition can also comprise more than one fatty alcohol such as any mixture of the above mentioned fatty alcohols. Preferred fatty alcohols are cetyl, stearyl, behenyl alcohols and mixtures thereof. Most preferred are stearyl alcohol, behenyl alcohol and cetylstearyl (or cetearyl) alcohol which is a mixture, usually 1 to 1 by weight, of cetyl and stearyl alcohol available for example from Cognis under the trade name Lanette O.

Concentration of fatty alcohol and in case more than one fatty alcohol is contained the total concentration of mixture of fatty alcohols, is in the range of 0.5 to 20%, preferably 1 to 15% and more preferably 2 to 10% by weight, calculated to total composition.

Compositions of the present invention comprise at least one emulsifier selected from cationic, non-ionic and amphoteric surfactants. Anionic surfactants are principally suitable emulsifiers but not preferred because of compatibility problems raise in the presence of cationic compounds and especially in the present invention with cationic polymers. Preferred surfactants as emulsifiers are cationic and non-ionic ones and mixtures thereof.

Cationic emulsifiers are any cationic surfactant available for cosmetic use. Preferably the cationic surfactants as emulsifiers are those mono alkyl quaternary ammonium compounds according to the general formula 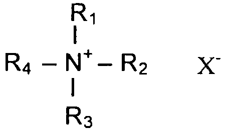 where R₁ is a saturated or unsaturated, branched or non-branched alkyl chain with 8-22 C atoms or

R₅CONH(CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Non-limiting examples to suitable cationic surfactants are such as cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, stear trimonium chloride, stearamidopropyldimethylammonium chloride and stearamidopropyl trimonuim chloride.

Suitable non-ionic surfactants are alkyl polyglucosides of the general formula

R₇-O-(R₈O)ₙO-Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

Among the non-ionic surfactants mentioned above fatty alcohol ethoxylates are the most preferred ones. Above mentioned non-ionic surfactants can also be used as mixture of one category such as several ethoxylated fatty alcohols or several categories such as mixture of alkyl polyglucoside and ethoxylated fatty alcohol.

As further surfactant component as emulsifier, the compositions according to the invention can also contain amphoteric or zwitterionic surfactants. Useful as such are the various known betaines such as alkyl betaines, fatty acid amidoalkyl betaines for example cocamido propyl betaine, and sulfobetaines, for example, lauryl hydroxysulfobetaine; long-chain alkyl amino acids, such as cocoaminoacetate, cocoaminopropionate and sodium cocoamphopropionate and -acetate.

Concentration of at least one emulsifier and in case more than one emulsifier is used the total emulsifier, is in the range of 0.1 to 15%, preferably 0.5 to 10% and more preferably 0.75 to 7.5% and most preferably 1 to 5% by weight calculated to total composition.

Compositions of the present invention can comprise additional hair conditioning compounds such as cationic surfactants, oils, cationic polymers, non-ionic substances. Oils as conditioners according to the present invention are selected from silicone oils either volatile or non-volatile, natural and synthetic oils. Among silicone oils those can be added to the compositions include dimethicone, dimethiconol, polydimethylsiloxane, DC fluid ranges from Dow Corning, cyclosiloxanes such as DC 245, arylated silicones such as phenyltrimethicone available from Dow Corning under trade name DC 556 and trimethyl pentaphenyl trisiloxane available under the trade name DC PH1555 HRI from Dow Corning.. Synthetic oils include mineral oil such as paraffin oil and petrolatum.

Natural oils suitable are such as olive oil, almond oil, avocado oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof.

Lipophilic oily compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₉CO(OCH₂CH₂)ₙOH

R₉CO(OCH₂CH₂)ₙOOCR₁₀

where R₉ and R₁₀ are independent from each other saturated, unsaturated or branched or non-branched alkyl chain with 7 to 21 C atoms and n is typically 2 - 100.

Composition of the present invention may comprise additional cationic polymers as conditioning agents. Suitable ones are such as cationic guar gums known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium. Typical examples of those Polyquaternium 6, Polyquaternium 7, Polyquaternium 11, Polyquaternium 16, Polyquaternium 22 and Polyquaternium 28, Polyquaternium 30, Polyquaternium 37, Polyquaternium 36, Polyquaternium 46,

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-81, Quaternium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

Cationic surfactant as conditioning agent selected from the compounds with the general formula 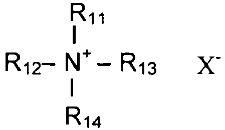 where R₁₁ and R₁₂ are independent from each other saturated or unsaturated, branched or non-branched alkyl chain with 8-22 C atoms or

R₁₅CO NH (CH₂)ₙ

where R₁₅ is saturated or unsaturated, branched or non-branched alkyl chain with 7-21 C atoms and n has typical value of 0 - 4 or

R₁₆CO O (CH₂)ₙ

where R₁₆ is saturated or unsaturated, branched or non-branched alkyl chain with 7-21 C atoms and n has typical value of 0 - 4, and
and R₁₃ and R₁₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

In principal any cationic surfactant according to the above general formula available for cosmetic purposes is suitable for the compositions of the present invention. Non-limiting examples to suitable cationic surfactants as conditioning agents are distearyl dimethyl ammonium chloride, distearoyl dimethyl ammonium chloride, dimethyl dihydrogenated tallow ammonium chloride, dipalmitoyl dimonium chloride, distearyl dimethyl ammonium chloride, distearamidopropyl dimonuim chloride.

Further examples to the cationic surfactants are so called esterquats available on the market, for example, under the trade names "Schercoquat^{®}", "Dehyquart^{®} L80" and "Tetranyl^{®}". Still further examples are so called amidoquats again available on the market, for example, under the trade name "INCROQUAT^{®} HO" or "OCS".

Concentration of any of the additional conditioners mentioned above either alone or in mixture with each other can be in the range of 0.01 to 15%, preferably 0.05 - 10%, more preferably 0.1 - 5% by weight, calculated to the total composition.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R}". Extracts and the preparation thereof are also described in "Hagers Handbuch der pharmazeutischen Praxis", 4^{th} Ed.

The compositions may contain one or more organic solvent such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, propylene glycol, 1-phenylethylalcohol, 2-phenylethylalcohol, o-methoxyphenol. Concentration of organic solvents in the composition should not exceed 15% by weight, preferably in the range of 1 to 10% by weight, calculated to total composition.

Compositions of the present invention can comprise one or more UV filter either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher and/or polysilicone-15. The preferred concentration of the UV-absorber ranges from about 0.01 % to 2.5%, more preferably from 0.05 % to 1 % by weight, calculated to the total composition.

The compositions of the present invention can comprise hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide.

Preferred fatty acids are those with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and especially 0.01 to 1% by weight calculated to the total weight of the composition. Phytosterol concentration of the conditioners is less than 1% and preferably in the range of 0.01 to 0.5% by weight calculated to the total weight of the composition. It should be noted without limiting the use of those ingredients the effect of those hair restructuring ingredients is especially elevated when used in combination with organic solvents as penetration enhancers.

In a further embodiment of the present invention compositions comprise at least one direct dye for colouring hair. Suitable direct dyes are cationic, anionic, neutral dyes and mixtures thereof as available commercially from various suppliers and used mainly in semi-permanent hair coloration.

One of the suitable direct dyes are cationic dyes. Non-limiting examples are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and their salts such as chloride, methosulfate, bromide etc. and mixtures thereof. Among the cationic direct dyes preferred are Basic red 51, Basic orange 31 and Basic yellow 87 and their mixture and/or their mixture with the remaining cationic dyes listed above.

Further suitable direct dyes are anionic dyes. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and their mixtures.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and their mixtures.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. In other words, cationic, anionic and nitro dyes are used in mixture within the meaning of the present invention. When using direct dyes of various categories, their compatibility must be taken into account.

Among the direct dyes cationic and nitro dyes are preferred ones. Most preferred ones are cationic direct dyes.

Concentration of direct dyes in the compositions of the present invention is within the range of 0.001 to 5%, preferably 0.01 to 4% and more preferably 0.05 to 3%, and most preferably 0.1 to 2% by weight, calculated to total composition.

Furthermore compositions of the present invention may comprise colour effect pigment consisting of synthetic mica coated with metal oxide or oxides and having a particle size distribution of 1 to 750 µm at a concentration of 0.01 to 10% by weight, calculated to total composition. When synthetic mica coated with metal oxide or oxides is present the preferred usage is rinse off.

The pH of the compositions according to the invention is in the range of 2 to 6, preferably 3 to 5, more preferably 3 to 4.5. For adjusting the pH of the said compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1.5% by weight calculated to the total composition. The pH of the composition can also be adjusted to the required pH by using alkaline solution such as sodium hydroxide, ammonium hydroxide, potassium hydroxide or their salts with those acids mentioned above in the case that at the selected acid concentration pH of the composition is lower than that of the aimed value.

Furthermore compositions of the present invention can comprise all substances customarily found in such preparations. Examples of such substances are complexing agents, preservatives, fragrances, etc.

Compositions of the present invention are suitable for use both as rinse off and leave in especially after cleansing hair. In case of leave in usage, use without previous cleansing is not excluded.

Accordingly, process for conditioning hair wherein hair is cleansed with a suitable cleansing composition as usual and subsequently a composition comprising at least one cationic polymer with multiple cationic sites and with multiple ester groups in its molecule, at least one cationic cellulose derivative and at least one cationic or cationizable silicone compound is applied onto hair and after processing of 30 sec to 30 min at a temperature of 20 to 45°C rinsed off from hair.

Further process for conditioning hair according to the present invention is application of a conditioning composition comprising at least one cationic polymer with multiple cationic sites and with multiple ester groups in its molecule, at least one cationic cellulose derivative and at least one cationic or cationizable silicone compound onto cleansed or wetted or dry hair and is not rinsed of from hair.

In a trial it has further been found out that hair conditioning properties of the compositions of the present invention is particularly elevated when hair cleansed with a cleansing composition comprising as well cationic polymer with multi cationic sites and ester groups, preferably polyquaternium 70 and a cationic cellusic polymer and cationic or cationizable silicone compopund. Accordingly, further subject of the present invention is kit for cleansing and conditioning hair which comprises a cleansing composition comprising at least one cationic polymer with multiple cationic sites and with multiple ester groups in its molecule, at least one cationic cellulose derivative and at least one cationic or cationizable silicone compound and a composition comprising at least one cationic polymer with multiple cationic sites and with multiple ester groups in its molecule, at least one cationic cellulose derivative and at least one cationic or cationizable silicone compound.

Following examples are to illustrate the invention but not to limit.

### Example 1

| | % by weight |
|---|---|
| Polyquaternium-70* | 0.25 |
| Polyquaternium-67 | 0.50 |
| Quaternium-80 | 0.75 |
| Hydroxyethylcellulose | 1.0 |
| Citric acid | q.s. to pH 4.5 |
| Fragrance, preservative | q.s. |
| Water | q.s. to 100 |

| | |
|---|---|
| *: used as Lustreplex, the concentration in the composition refers to active matter. | |

The above composition can be used as a rinse off composition and also as a leave-in composition. Under both usage condition it was observed that the above composition improves combability, gives hair shine, especially in rinse off usage and give hair volume and body (body is very much enhanced especially in leave-in usage) and makes hair manageable. It was also observed that hair is possible to style easily and style holds reasonably for a long period of time especially under leave-in usage.

### Example 2

| | % by weight |
|---|---|
| Stearyl alcohol | 7.0 |
| Cetrimonium chloride | 1.0 |
| Polyquaternium-70* | 0.5 |
| Polyquaternium-10 | 0.75 |
| Quaternium-80 | 0.5 |
| Citric acid/Sodium hydroxide | q.s. to pH 4.0 |
| Fragrance | 0.2 |
| Water | to 100 |

| | |
|---|---|
| *: used as Lustreplex, the concentration in the composition refers to active matter. | |

The above composition was prepared by emulsifying stearyl alcohol and cetrimonium chloride at approximately 70°C in part of water and subsequently after cooling down to around 40°C polyquaternium 70 (Lustreplex), solution of polyquaternium 10 in water, Quaternium-80 and fragrance were added. Finally pH was adjusted.

For comparative purpose the above composition was prepared by replacing the polyquaternium 70 with the same amount of polyquaternium 10.

The above composition was tested in a half side test with 10 consumers having shoulder length damaged hair against the comparative composition. Before application of the above composition, hair was washed with a commercially available shampoo. Afterwards to the half side 8 g of the above composition of Example 1 and comparative composition were applied and processed for 5 min at ambient temperature. At the end of the processing time the hair was rinsed off with tap water at approximately 40°C. The hair was towel dried and dried with a hair drier. The hair was evaluated in wet (before drying) and dry states by hair dressers (at least 2) and customers preference was asked. The following results were obtained.

**Table I: Results of half side comparative test of Example I**

| | Example 1 | Comparative composition | No difference |
|---|---|---|---|
| Wet hair | | | |
| Combability | 6 | 3 | 1 |
| Smoothness | 7 | 3 | 0 |
| Roughness | 0 | 1 | 9 |
| Dry hair | | | |
| Combability | 7 | 2 | 1 |
| Smoothness | 7 | 2 | 1 |
| Roughness | 1 | 2 | 7 |
| Shine | 7 | 2 | 1 |
| Volume | 6 | 2 | 2 |
| Body | 5 | 3 | 2 |
| Elasticity | 5 | 2 | 3 |
| Preference | 6 | 2 | 2 |

From the above results it is clear that conditioning properties of the inventive composition is much better than the comparative composition.

### Example 3

| | % by weight |
|---|---|
| Cetearyl alcohol | 6.0 |
| Cteareth-20 | 2.0 |
| Polyquaternium-70* | 0.4 |
| Polyquaternium-24 | 0.8 |
| Amodimethicone** | 1.0 |
| Citric acid/Sodium hydroxide | q.s. to pH 4.0 |
| Fragrance | 0.2 |
| Water | to 100 |

| | |
|---|---|
| *: used as Lustreplex, the concentration in the composition refers to active matter. ** DC 949, the number represents amodimethicone active matter | |

The above composition was prepared in the same way as the example 1. The above composition is suitable for leave-in and rinse of usages.

Upon use on fine hair it was found out that hair is excellently combable and feels smooth, has improved shine, elasticity and volume and body.

### Example 4

| | % by weight |
|---|---|
| Cetearyl alcohol | 8.0 |
| Cteareth-20 | 3.0 |
| Stearamidopropyl trimonuim chloride | 1.0 |
| Quaternium-80 | 0.5 |
| Polyquaternium-10 | 0.1 |
| Polyquaternium-70* | 0.4 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. | |

### Example 5

| | % by weight |
|---|---|
| Behenyl alcohol | 9.0 |
| Behentrimonuim chloride | 2.0 |
| Polyquaternium-70* | 0.4 |
| Quaternium-80 | 0.5 |
| Polyquaternium-10 | 0.1 |
| Citric acid/Sodium hydroxide | q.s. to pH 4.0 |
| Fragrance | 0.2 |
| Water | to 100 |

| | |
|---|---|
| *: used as Lustreplex, the concentration in the composition refers to active matter. | |

### Example 6

| | % by weight |
|---|---|
| Behenyl alcohol | 7.0 |
| Behentrimonuim chloride | 1.0 |
| Ceteareth-20 | 1.0 |
| Polyquaternium-70* | 0.4 |
| Quaternium-80 | 0.3 |
| Polyquaternium-24 | 0.3 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. | |

### Example 7

| | % by weight |
|---|---|
| Behenyl alcohol | 8.0 |
| Behentrimonuim chloride | 1.5 |
| Ceteareth-20 | 0.8 |
| Polyquaternium-70* | 0.4 |
| Quaternium-80 | 0.3 |
| Polyquaternium-67 | 0.3 |
| Benzophenone-3 | 0.3 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. | |

### Example 8

| | % by weight |
|---|---|
| Cetearyl alcohol | 10.0 |
| Behentrimonuim chloride | 1.0 |
| Ceteareth-20 | 1.0 |
| Polyquaternium-70* | 0.4 |
| Polysiliocne-9 | 0.3 |
| Polyquaternium-4 | 0.3 |
| Dimethicone** | 1.0 |
| Benzophenone-3 | 0.3 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. **: DC 200 with a viscosity of 60,000 mPa.s. | |

### Example 9

| | % by weight |
|---|---|
| Polyqauternium-37 | 1.5 |
| Polyquaternium-70* | 0.4 |
| Amodimethicone** | 0,5 |
| Polyquaternium-10 | 0.2 |
| Benzophenone-3 | 0.3 |
| Ethanol | 5.0 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. **: Belsil ADM 8020, the concentration refers to active amodimethicone concentration | |

### Example 10

| | % by weight |
|---|---|
| Cetearyl alcohol | 9.0 |
| Behentrimonium chloride | 1.5 |
| Ceteareth-20 | 3.0 |
| Polyquaternium-70* | 0.4 |
| Quaternium-80 | 0.3 |
| Polyquaternium-67 | 0.3 |
| Polysilicone-15 | 0.3 |
| Basic red 51 | 0.1 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. | |

### Example 11

| | % by weight |
|---|---|
| Cetearyl alcohol | 9.0 |
| Behentrimonium chloride | 1.5 |
| Ceteareth-20 | 3.0 |
| Polyquaternium-70* | 0.4 |
| Quaternium-80 | 0.3 |
| Polyquaternium-67 | 0.3 |
| Polysilicone-15 | 0.3 |
| Benzophenone-3 | 0.3 |
| Basic red 51 | 0.1 |
| Basic red 76 | 0.1 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. | |

### Example 12

| | % by weight |
|---|---|
| Cetearyl alcohol | 9.0 |
| Behentrimonium chloride | 1.5 |
| Ceteareth-20 | 3.0 |
| Polyquaternium-70* | 0.4 |
| Polysiliocne-9 | 0.3 |
| Polyquaternium-67 | 0.3 |
| Polysilicone-15 | 0.3 |
| Benzophenone-3 | 0.3 |
| Basic orange 31 | 0.04 |
| Basic yellow 87 | 0.1 |
| Citric acid/Sodium hydroxide | q.s. to pH 3.5 |
| Fragrance | 0.2 |
| Water | to 100 |

| | |
|---|---|
| *: used as Lustreplex, the concentration in the composition refers to active matter. | |

## Claims

1. An aqueous composition for conditioning keratin fibres especially human hair **characterised in that** it comprises at least one cationic polymer with multiple cationic sites and with multiple ester groups in its molecule, at least one cationic cellulose derivative and at least one cationic or cationizable silicone compound.

2. Composition according to claim 1 **characterised in that** the cationic polymer with multiple cationic sites and with multiple ester groups in its molecule is Polyquaternium-70.

3. Composition according to claims 1 and 2 at least one cationic cellulosic polymer is chosen from Polyqauternium 4, Polyqauternium 10, Polyqauternium 24, Polyqauternium 67 and Polyqauternium 72.

4. Composition according to any of the preceding claims **characterised in that** at least one cationizable or cationic silicone compound is chosen from amodimethicone, quaternium-80 and quaternised graft polymers of organopolysiloxane and polyethyloxazoline.

5. Composition according to any of the preceding claims **characterised in that** it is an emulsion or a thickened gel.

6. Composition according to any of the preceding claims it comprises at least one fatty alcohol and at least one emulsifier selected from cationic, non-ionic and amphoteric surfactants.

7. Composition according to claim 6 **characterised in that** at least one emulsifier is a cationic surfactant of following general formula 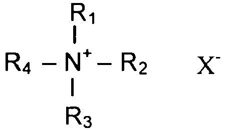 where R₁ is a saturated or unsaturated, branched or non-branched alkyl chain with 8-22 C atoms or
R₅CONH(CH₂)ₙ
where R₅ is saturated or unsaturated, branched or non-branched alkyl chain with 7-21 C atoms and n has typical value of 0 - 4 or
R₆COO(CH₂)ₙ
where R₆ is saturated or unsaturated, branched or non-branched alkyl chain with 7-21 C atoms and n has typical value of 0 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

8. Composition according to claims 6 and 7 **characterised in that** at least one emulsifying surfactant is non-ionic surfactant and selected from alkyl polyglucosides of the general formula
R₇-O-(R₈O)ₙO-Zₓ
wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5, long chain fatty acid mono or dialkanolmaides, sorbitan esters and C₁₀-C₂₂-fatty alcohol ethoxylates.

9. Composition according to any of the preceding claims **characterised in that** it comprises at least one organic solvent

10. Composition according to any of the preceding claims **characterised in that** it comprises at least one UV filter.

11. Composition according to any of the preceding claims **characterised in that** it comprises at least one direct dye.

12. Use of compositions according to claims 1 to 11 for conditioning hair.

13. Process for conditioning hair wherein hair is cleansed with a suitable cleansing composition as usual and subsequently a composition according to claims 1 to 11 is applied onto hair and after processing of 30 sec to 30 min at a temperature of 20 to 45°C rinsed off from hair.

14. Process for conditioning hair wherein a composition according to claims 1 to 11 is applied onto cleansed or wetted or dry hair and without rinsing off hair is dried.

15. Kit for cleansing and conditioning hair **characterised in that** it comprises a cleansing composition comprising at least one cationic polymer with multiple cationic sites and with multiple ester groups in its molecule, at least one cationic cellulose derivative and at least one cationic or cationizable silicone compound and a conditioning composition according to claims 1 to 11.

## Patentansprüche

1. Wässrige Zusammensetzung zum Konditionieren von Keratinfasern, insbesondere menschlichem Haar, **dadurch gekennzeichnet, dass** sie mindestens ein kationisches Polymer mit mehreren kationischen Stellen und mit mehreren Estergruppen in seinem Molekül, mindestens ein kationisches Cellulosederivat und mindestens eine kationische oder kationisierbare Silikonverbindung aufweist.

2. Zusammensetzung nach Anspruch 1, **dadurch gekennzeichnet, dass** das kationische Polymer mit mehreren kationischen Stellen und mehreren Estergruppen in seinem Molekül Polyquaternium 70 ist.

3. Zusammensetzung nach Anspruch 1 und 2, **dadurch gekennzeichnet** mindestens ein kationisches cellulosisches Polymer ist aus Polyquaternium 4, Polyquaternium 10, Polyquaternium 24, Polyquaternium 67 und Polyquaternium 72 ausgewählt.

4. Zusammensetzung nach einem der vorhergehenden Ansprüche, **dadurch gekennzeichnet, dass** mindestens eine kationische oder kationisierbare Silikonverbindung aus Amodimethicon, Quaternium 80 und quaternisierten Pfropfpolymeren von Organopolysiloxan und Polyethyloxazolin ausgewählt ist.

5. Zusammensetzung nach einem der vorhergehenden Ansprüche, **dadurch gekennzeichnet, dass** sie eine Emulsion oder ein verdicktes Gel ist.

6. Zusammensetzung nach einem der vorhergehenden Ansprüche, **dadurch gekennzeichnet** sie weist mindestens einen Fettalkohol und mindestens einen Emulgator auf, der aus kationischen, nichtionischen und amphoteren Tensiden ausgewählt ist.

7. Zusammensetzung nach Anspruch 6, **dadurch gekennzeichnet, dass** mindestens ein Emulgator ein kationisches Tensid der folgenden allgemeinen Formel 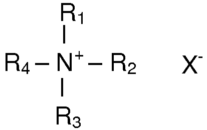 ist, wobei R₁ für eine gesättigte oder ungesättigte, verzweigte oder unverzweigte Alkylkette mit 8 bis 22 C-Atomen steht oder für
R₅CONH(CH₂)ₙ,
wobei R₅ für eine gesättigte oder ungesättigte, verzweigte oder unverzweigte Alkylkette mit 7 bis 21 C-Atomen steht und n einen typischen Wert von 0 bis 4 aufweist, oder für
R₆COO(CH₂)ₙ,
wobei R₆ für eine gesättigte oder ungesättigte, verzweigte oder unverzweigte Alkylkette mit 7 bis 21 C-Atomen steht und n einen typischen Wert von 0 bis 4 aufweist, und
R₂, R₃ und R₄ unabhängig voneinander für H oder eine niedere Alkylkette mit 1 bis 4 Kohlenstoffatomen oder eine Ethoxy- oder Propoxygruppe steht, wobei die Anzahl der Ethoxy- oder Propoxygruppe im Bereich von 0 bis 4 variiert und X für Chlorid, Bromid oder Methosulfat steht.

8. Zusammensetzung nach Anspruch 6 und 7, **dadurch gekennzeichnet, dass** mindestens ein emulgierendes Tensid ein nichtionisches Tensid ist und aus Alkylpolyglucosiden der allgemeinen Formel
R₇-O-(R₈O)ₙO-Zₓ,
wobei R₇ für eine Alkylgruppe mit 8 bis 18 Kohlenstoffatomen steht, R₈ für eine Ethylen- oder Propylengruppe steht, Z für eine Saccharidgruppe mit 5 bis 6 Kohlenstoffatomen steht, n eine Zahl von 0 bis 10 ist und x eine Zahl von 1 bis 5 ist, langkettigen Fettsäure-mono- oder dialkanolamiden, Sorbitanestern und C₁₀-₂₂-Fettalkoholethoxylaten ausgewählt ist.

9. Zusammensetzung nach einem der vorhergehenden Ansprüche, **dadurch gekennzeichnet, dass** sie mindestens ein organisches Lösungsmittel aufweist.

10. Zusammensetzung nach einem der vorhergehenden Ansprüche, **dadurch gekennzeichnet, dass** sie mindestens einen UV-Filter aufweist.

11. Zusammensetzung nach einem der vorhergehenden Ansprüche, **dadurch gekennzeichnet, dass** sie mindestens einen Direktfarbstoff aufweist.

12. Verwendung von Zusammensetzungen nach Anspruch 1 bis 11 zum Konditionieren von Haar.

13. Verfahren zum Konditionieren von Haar, wobei das Haar wie üblich mit einer geeigneten Reinigungszusammensetzung gereinigt wird und anschließend eine Zusammensetzung nach Anspruch 1 bis 11 auf das Haar aufgebracht wird und nach einem Einwirken von 30 Sekunden bis 30 Minuten bei einer Temperatur von 20 °C bis 45 °C aus dem Haar ausgespült wird.

14. Verfahren zum Konditionieren von Haar, wobei eine Zusammensetzung nach Anspruch 1 bis 11 auf gereinigtes oder befeuchtetes oder trockenes Haar aufgebracht wird und das Haar ohne Ausspülen getrocknet wird.

15. Kit zum Reinigen und Konditionieren von Haar, **dadurch gekennzeichnet, dass** es eine Reinigungszusammensetzung, welche mindestens ein kationisches Polymer mit mehreren kationischen Stellen und mit mehreren Estergruppen in seinem Molekül, mindestens ein kationisches Cellulosederivat und mindestens eine kationische oder kationisierbare Silikonverbindung aufweist, und eine Konditionierungszusammensetzung nach Anspruch 1 bis 11 aufweist.

## Revendications

1. Composition aqueuse pour le conditionnement de fibres de kératine, notamment de cheveux humains, **caractérisée en ce qu'**elle comprend au moins un polymère cationique avec de multiples sites cationiques et avec de multiples groupements ester dans sa molécule, au moins un dérivé cationique de cellulose et au moins un composé de silicone cationique ou pouvant être rendu cationique.

2. Composition selon la revendication 1, **caractérisée en ce que** le polymère cationique avec de multiples sites cationiques et avec de multiples groupements ester dans sa molécule est du Polyquaternium-70.

3. Composition selon les revendications 1 et 2, **caractérisée en ce que** au moins un polymère cellulosique cationique est choisi parmi le Polyquaternium 4, le Polyquaternium 10, le Polyquaternium 24, le Polyquaternium 67 et le Polyquaternium 72.

4. Composition selon l'une quelconque des revendications précédentes, **caractérisée en ce qu'**au moins un composé de silicone cationique ou pouvant être rendu cationique est choisi parmi l'amodiméthicone, le quaternium-80 et des polymères quaternisés greffés d'organopolysiloxane et de polyéthyloxazoline.

5. Composition selon l'une quelconque des revendications précédentes, **caractérisée en ce qu'**elle est une émulsion ou un gel épaissi.

6. Composition selon l'une quelconque des revendications précédentes, **caractérisée en ce que** elle comprend au moins un alcool gras et au moins un émulsifiant choisi parmi les tensioactifs cationiques, non ioniques et amphotères.

7. Composition selon la revendication 6, **caractérisée en ce qu'**au moins un émulsifiant est un tensioactif cationique selon la formule générale suivante : 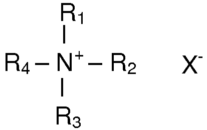 où R₁ est une chaîne alkyle saturée ou non saturée, ramifiée ou non ramifiée, avec 8 à 22 atomes C ou un groupement
R₅CONH(CH₂)ₙ
où R₅ est une chaîne alkyle saturée ou non saturée, ramifiée ou non ramifiée, avec 7 à 21 atomes C et n a une valeur typique de 0 à 4, ou un groupement
R₆COO(CH₂)ₙ
où R₆ est une chaîne alkyle saturée ou non saturée, ramifiée ou non ramifiée, avec 7 à 21 atomes C et n a une valeur typique de 0 à 4, et
R₂, R₃ et R₄ sont indépendamment les uns des autres un atome H ou une chaîne alkyle plus courte avec 1 à 4 atomes de carbone, ou un groupement éthoxy ou propoxy, avec un nombre de groupements éthoxy ou propoxy variant dans une plage de 0 à 4, et X est un ion chlorure, bromure ou méthosulfate.

8. Composition selon les revendications 6 et 7, **caractérisée en ce qu'**au moins un tensioactif émulsifiant est un tensioactif non ionique et est choisi parmi les alkylpolyglucosides selon la formule générale
R₇-O-(R₈O)ₙO-Zₓ
où R₇ est un groupement alkyle avec 8 à 18 atomes de carbone, R₈ est un groupement éthylène ou propylène, Z est un groupement saccharide avec 5 à 6 atomes de carbone, n est un nombre entre 0 et 10 et x est un nombre entre 1 et 5, un mono ou un dialcanolamide d'acide gras à longue chaîne, des esters de sorbitane et des éthoxylates d'alcool gras en C₁₀ à C₂₂.

9. Composition selon l'une quelconque des revendications précédentes, **caractérisée en ce qu'**elle comprend au moins un solvant organique.

10. Composition selon l'une quelconque des revendications précédentes, **caractérisée en ce qu'**elle comprend au moins un filtre UV.

11. Composition selon l'une quelconque des revendications précédentes, **caractérisée en ce qu'**elle comprend au moins un colorant direct.

12. Utilisation de compositions selon les revendications 1 à 11 pour le conditionnement des cheveux.

13. Traitement pour le conditionnement des cheveux dans lequel les cheveux sont nettoyés avec une composition de nettoyage appropriée comme d'habitude, et ensuite une composition selon les revendications 1 à 11 est appliquée sur les cheveux et est éliminée par rinçage des cheveux après un traitement de 30 secondes à 30 minutes à une température de 20 à 45 °C.

14. Traitement pour le conditionnement des cheveux dans lequel une composition selon les revendications 1 à 11 est appliquée sur les cheveux nettoyés ou humides ou secs, et est séchée sans rinçage des cheveux.

15. Kit pour le nettoyage et le conditionnement des cheveux **caractérisé en ce qu'**il comprend une composition nettoyante comprenant au moins un polymère cationique avec de multiples sites cationiques et avec de multiples groupements ester dans sa molécule, au moins un dérivé cationique de cellulose et au moins un composé de silicone cationique ou pouvant être rendu cationique et une composition de conditionnement selon les revendications 1 à 11.
